(19) Europäisches Patentamt
European Patent Office
Office européen des brevets

(11) Numéro de publication: **0 272 976 B1**

(12) **FASCICULE DE BREVET EUROPEEN**

(45) Date de publication de fascicule du brevet: **04.09.91**

(51) Int. Cl.5: **A61K 45/06**, A61K 31/565, A61K 31/55, A61K 31/135, //(A61K31/565,31:55,31:135)

(21) Numéro de dépôt: **87402824.4**

(22) Date de dépôt: **11.12.87**

(54) **Compositions zootechniques renfermant un béta adrénergique.**

(30) Priorité: **11.12.86 FR 8617334**
**13.08.87 FR 8711543**
**13.08.87 FR 8711542**

(43) Date de publication de la demande:
**29.06.88 Bulletin 88/26**

(45) Mention de la délivrance du brevet:
**04.09.91 Bulletin 91/36**

(84) Etats contractants désignés:
**AT BE CH DE FR GB IT LI LU NL SE**

(56) Documents cités:
**EP-A- 0 107 569**
**FR-A- 2 230 378**
**FR-A- 2 238 476**
**FR-A- 2 388 560**
**GB-A- 1 392 889**

**CHEMICAL ABSTRACTS, vol. 106, no. 5, 2 février 1987, page 441, résumé 31792w, Columbus, Ohio, US; O. BOHOROV et al.: "The effect of the bêta2-adrenergic agonist clenbuterol or implantation with estradiol plus trenbolone acetate on protein metabolism in wether lambs"**

(73) Titulaire: **ROUSSEL-UCLAF**
**35, boulevard des Invalides**
**F-75007 Paris(FR)**

(72) Inventeur: **Grandadam, Jean André**
**4, rue Lhomme**
**F-94100 Saint Maur des Fosses(FR)**

(74) Mandataire: **Tonnellier, Marie-José et al**
**111, route de Noisy B.P. no 9**
**F-93230 Romainville(FR)**

**EP 0 272 976 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles compositions zootechniques renfermant un béta adrénergique.

L'invention a pour objet les associations caractérisées en ce qu'elles renferment :

a) une composition zootechnique renfermant un béta adrénergique,

b) une composition zootechnique renfermant comme principe actif, un stéroïde de formula (A) :

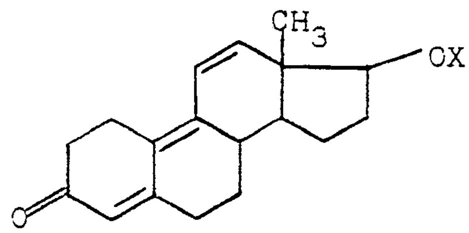

(A)

dans laquelle X représente un atome d'hydrogène, un radical alcoyle saturé ou insaturé renfermant de 1 à 6 atomes de carbone dont l'un des atomes de carbone peut être remplacé par un atome d'oxygène ou X représente un radical acyle dérivé d'un acide carboxylique renfermant de 1 à 18 atomes de carbone, pour une utilisation simultanée, séparée ou étalée dans le temps.

Les béta adrénergiques sont une famille de médicaments bien connue en médecine humaine. Certains de ces béta adrénergiques ont été décrits comme présentant des propriétés anabolisantes chez l'animal (voir à ce sujet, les brevets DE 3234995 et EP 103830).

On vient de découvrir que l'on obtient des résultats particulièrement intéressants en associant les béta adrénergiques aux produits de formule (A). Les associations de l'invention permettent d'améliorer de facon remarquable la prise de poids et la qualité de viande chez les animaux d'élevage comme les bovins, les porcins, les ovins ou les poulets, comme le montrent les résultats d'essais exposés ci-après dans la partie expérimentale.

L'effet obtenu en associant les béta adrénergiques et les produits de formule (A) est bien supérieur à celui de la somme des effets obtenus en administrant les béta adrénergiques seuls et les produits de formule (A) seuls.

Les produits de formule (A) sont des produits connus décrits notamment dans les brevets francais 1.380.414 et 1.492.985 ainsi que dans le brevet belge 696.084.

Parmi les produits de formule (A) préférés, on peut citer le 3-oxo 17-béta-acétoxy estra 4,9,11-triène.

Parmi les associations préférées de l'invention, on peut citer les associations caractérisées en ce que le stéroïde de formule (A) et l'acétate de trenbolone ou 3-oxo 17béta-acétoxy estra 4,9,11-triène ainsi que celles caractérisées en ce qu'elles renferment comme béta-adrénergique au moins un composé de formule générale (I) :

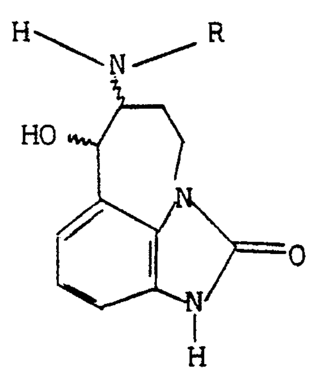

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventeullement un substituant alcoyle renfermant de 1 à 4 atomes de carbone et les traits ondulés signifient que l'hydroxyle en position 7 et le radical amino en position 6

sont de configuration trans ou un de ses sels d'addition avec les acides minéraux ou organiques.

Dans la formule générale (I) et dans ce qui suit, le terme radical alcoyle, linèaire ou ramifié, renfermant de 1 à 8 atomes de carbone, désigne, par exemple, un radical n-pentyle, n-butyle, n-propyle, 2,2-diméthylpropyle ou de préférence, un radical isopropyle, éthyle ou méthyle ;

le terme radical cycloalcoyle renfermant de 3 à 7 atomes de carbone désigne un radical cyclopropyle, cyclobutyle, cyclopentyle, cyclohexyle ou cycloheptyle ;

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, tels que les acides méthane ou éthane sulfoniques, arylsulfoniques, tels que les acides benzène ou paratoluène sulfoniques et arylcarboxyliques.

Les produits de formule (I) sont des produits connus pour leur activité antihypertensive et hypotensive (voir à ce sujet, le brevet européen 0107569).

L'invention a notamment pour objet les associations renfermant un produit répondant à la formule (I) dans laquelle R représente un atome d'hydrogène ou un radical alcoyle renfermant de 1 à 5 atomes de carbone ainsi que leurs sels d'addition avec les acides minéraux ou organiques.

L'invention a plus particulièrement pour objet les associations renfermant comme béta adrénergique, la (6RS,trans) 6-(1-méthyléthyl) amino 7-hydroxy 4,5,6,7-tétrahydro imidazo [5,4,1-j-k] [1] benzazépin-2-(1H)-one ainsi que ses sels d'addition avec les acides minéraux ou organiques et notamment son chlorhydrate.

Ce produit est décrit à l'exemple 14 du brevet précité.

Parmi les associations préférées de l'invention, on peut citer également les associations caractérisées en ce qu'elles renfermant comme béta adrénergique, le salbutamol de formule :

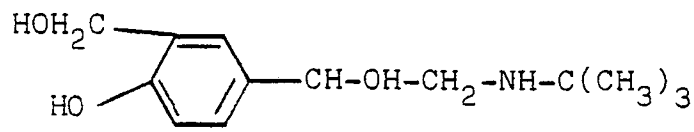

Le salbutamol est un produit connu : le salbutamol ou albutérol est le alpha [(tert-butylamino méthyl] 4-hydroxy n-xylène-alpha, alpha'- diol connu pour son activité sur les béta 2-récepteurs du muscle lisse bronchique et commercialisé comme médicament pour le traitement de l'asthme : voir à ce sujet, la rubrique 206 de la neuvième édition du Merck Index ou les rubriques "VENTOLINE", pages 1585, 1586, 1587 du dictionnaire VIDAL, 61ème édition (1985), par exemple.

Comme béta adrénergique, on peut encore citer le cimatérol, produit de formule :

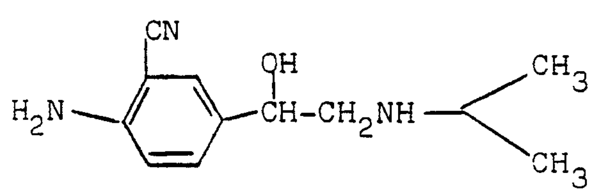

le clembutérol, produit de formule :

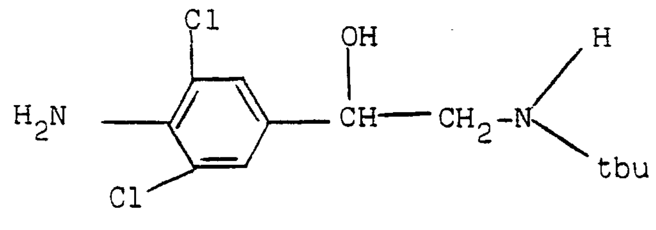

ainsi que les produits décrits dans les brevets déjà cités (DE 3.234.995 et EP 103.830).

L'invention a particulièrement pour objet les associations caractérisées en ce qu'elles renferment :

a) une composition zootechnique renfermant un béta adrénergique,

b) une composition zootechnique renfermant un produit de formule (A) telle que définie ci-dessus,

c) une composition zootechnique renfermant du zéranol, pour une utilisation simultanée, séparée ou étalée dans le temps et également les associations renfermant :

a) une composition zootechnique renfermant un béta adrénergique,

b) une composition zootechnique renfermant un produit de formule (A),

c) une composition zootechnique renfermant de l'estradiol, pour une utilisation simultanée, séparée ou étalée dans le temps.

On a trouvé que l'on obtenait des résultats particulièrement intéressants, en utilisant des associations renfermant:

a) une composition zootechnique renfermant comme principe actif, un produit de formule (I) défini précédemment,

b) une composition zootechnique renfermant comme principe actif un produit de formule (A) défini comme précédemment,

c) une composition zootechnique renfermant comme principe actif du zéranol,

pour une utilisation simultanée, séparée ou étalée dans le temps, ainsi que les associations renfermant :

a) une composition zootechnique renfermant comme principe actif un produit de formule I défini précédemment,

b) une composition zootechnique renfermant comme principe actif un produit de formule A défini précédemment,

c) une composition zootechnique renfermant comme principe actif de l'estradiol,

pour une utilisation simultanée, séparée ou étalée dans le temps.

Les résultats obtenus sont tout à fait remarquables, lorsque le produit de formule I utilisé est la (6RS, trans) 6-(1-méthyléthyl) amino 7-hydroxy 4,5,6,7-tétrahydro imidazo /5,4,1-j-k/ /1/ benzazépin-2-(1H) one, ou l'un de ses sels d'addition avec les acides organiques ou minéraux, et le produit de formule A est le 3-oxo 17 béta-acétoxy estra 4,9,11-triène.

L'invention a également pour objet les associations renfermant:

a) une composition zootechnique renfermant du salbutamol,

b) une composition zootechnique renfermant de l'acétate de trenbolone,

c) une composition zootechnique renfermant du zéranol pour une utilisation simultanée, séparée ou étalée dans le temps, ainsi que les associations renfermant :

a) une composition zootechnique renfermant du salbutamol,

b) une composition zootechnique renfermant de l'acétate de trenbolone,

c) une composition zootechnique renfermant de l'estradiol, pour une utilisation simultanée, séparée ou étalée dans le temps.

Comme association particulière de l'invention, on peut citer des associations caractérisées en ce qu'elles renferment :

a) une composition zootechnique renfermant comme principe actif, un produit de formule (I) défini précédemment,

b) une composition zootechnique renfermant comme principes actifs :

$b_1$) un produit de formule (A) défini comme précédemment,

$b_2$) du zéranol ou de l'estradiol,

pour une utilisation simultanée ou étalée dans le temps et notamment des associations renfermant :

a) une composition zootechnique renfermant comme principe actif la (6RS,trans) 6-(1-méthyléthyl) amino 7-hydroxy 4,5,6,7-tétrahydro imidazo /5,4,1-j-k/ /1/ benzazépin-2-(1H)-one ou l'un de ses sels d'addition avec les acides organiques ou minéraux,

b) une composition zootechnique renfermant comme principes actifs :

$b_1$) l'acétate de trenbolone,

$b_2$) le zéranol.

pour une utilisation simultanée, séparée ou étalée dans le temps.

Comme association particulière de l'invention, on peut également citer des associations caractérisées en ce qu'elles renferment :

a) une composition zootechnique renfermant du salbutamol,

b) une composition zootechnique renfermant :

$b_1$) de l'acétate de trenbolone, et

$b_2$) du zéranol,

pour une utilisation simultanée, séparée ou étalée dans le temps ainsi que les associations renfermant :

a) une composition zootechnique renfermant du salbutamol,

b) une composition zootechnique renfermant :

$b_1$) de l'acétate de trenbolone, et

$b_2$) de l'estradiol,

pour une utilisation simultanée, séparée ou étalée dans le temps.

L'invention a encore pour objet les compositions zootechniques renfermant comme principe actif au moins un composé de formule générale (I) :

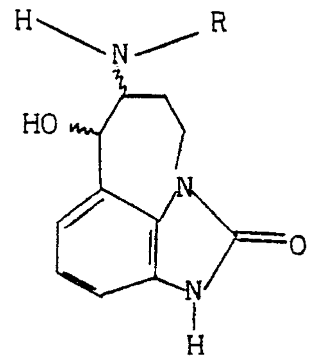

(I)

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans ou un de ses sels d'addition avec les acides minéraux ou organiques.

Dans la formule générale (I), comme il a été indiqué ci-dessus, le terme radical alcoyle, linéaire ou ramifié, renfermant de 1 à 8 atomes de carbone, désigne, par exemple, un radical n-pentyle, n-butyle, n-propyle, 2,2-diméthylpropyle ou de préférence, un radical isopropyle, éthyle ou méthyle ;

le terme radical cycloalcoyle renfermant de 3 à 7 atomes de caarbone désigne un radical cyclopropyle, cyclobutyle, cyclopentyle, cyclohexyle ou cycloheptyle ;

les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques, tels que les acides méthane ou éthane sulfoniques, arylsulfoniques, tels que les acides benzène ou paratoluène sulfoniques et arylcarboxyliques.

Parmi les composés préférés, on peut citer les composés répondant à la formule (I) dans laquelle R représente un atome d'hydrogène ou un radical alcoyle renfermant de 1 à 5 atomes de carbone ainsi que leurs sels d'addition avec les acides minéraux ou organiques.

L'invention a plus particulièrement pour objet les compositions zootechniques renfermant comme principe actif la (6RS,trans) 6-(1-méthyléthyl) amino 7-hydroxy 4,5,6,7-tétrahydro imidazo [5,4,1-j-k][1] benzazépin-2-(1H)-one ainsi que ses sels d'addition avec les acides minéraux ou organiques et notamment son chlorhydrate.

L'invention a aussi particulièrement pour objet les compositions renfermant comme principe actif du salbutamol. Les associations et les compositions zootechniques selon l'invention permettent d'améliorer la prise de poids et la qualité de la viande chez les animaux d'élevage tels que les bovins, les ovins, notamment les agneaux, les porcins ou les volailles.

L'invention a ainsi pour objet les associations définies ci-dessus destinées à l'administration chez le porc et celles destinées à l'administration chez le veau.

L'invention a également pour objet les compositions zootechniques définies ci-dessus destinées à l'administration chez le porc et celles destinées à l'administration chez le veau.

L'invention a enfin pour objet les associations et compositions zootechniques définies ci-dessus destinées à l'administration chez la volaille.

Les béta adrénergiques sont de préférence administrés aux animaux par voie orale, sous forme de comprimés, de granulés ou de poudres distribués dans la nourriture et préparés selon les procédés classiques de fabrication de tels produits. Les béta adrénergiques peuvent être administrés par voie parentérale.

On peut également incorporer les béta adrénergiques dans des compositions alimentaires en association avec un mélange nutritiel adapté à l'alimentation animale.

Le mélange nutritiel peut varier selon l'espèce animale, il peut renfermer des céréales, des sucres, des graines de tourteaux de soja, d'arachide et de tournesol, des farines d'origine animale, par exemple, des farines de poissons des acides aminés de synthèse, des sels minéraux, des vitamines et des anti-oxydants.

En vue de leur administration au bétail, les produits de formule (A) le zéranol et l'estradiol peuvent être déposés sous forme d'implant à la base de l'oreille ou au fanon.

Ils sont déposés, par exemple, de 20 jours à 4 mois avant l'abattage, de préférence de 1 à 3 mois avant celui-ci.

Les produits de formule (A), le zéranol et l'estradiol, peuvent également être soit injectés sous forme de solutions ou de suspensions, soit ingérés.

Les compositions ou associations de l'invention manifestent ainsi d'intéressantes propriétés anabolisantes et plus particulièrement des propriétés anabolisantes protidiques.

Ces propriétés rendent les associations ou compositions zootechniques de l'invention aptes à être utilisées comme médicaments vétérinaires, notamment pour augmenter la résistance générale organique aux agressions de toutes sortes, pour lutter contre les retards de croissance, l'amaigrissement, les troubles organiques généraux liés à un état de sénescence et également pour lutter à titre secondaire contre les maladies infectieuses, parasitaires et nutritionnelles.

La posologie usuelle peut être, par exemple, de 10 à 1000 mcg de béta adrénergique par kg de poids animal et par jour administrée par voie orale. Les béta adrénergiques peuvent être administrés sous forme de compositions zootechniques renfermant de 0,5 à 300 mg de béta adrénergique, sous forme d'implant, par exemple.

Le zéranol peut être administré sous forme de compositions zootechniques renfermant de 10 à 100 mg de zéranol, sous forme d'implant, par exemple. L'estradiol peut être administré sous forme de compositions zootechniques renfermant de 0,05 à 50 mg d'estradiol.

Le produit de formule A peut être administré sous forme de compositions zootechniques renfermant de 1 à 300 mg de produit A.

On peut citer comme particulièrement intéressante, l'association des compositions renfermant de 1 à 100 mg de béta adrénergique, par exemple, de 5 à 25 mg de béta adrénergique et d'implants renfermant de 5 à 50 mg d'acétate de trenbolone (ou 3-oxo 17-béta-acétoxy estra 4,9,11-triène) et de 10 à 50 mg de zéranol.

Les exemples suivants illustrent l'invention sans toutefois la limiter.

Dans les exemples suivants, le chlorhydrate de (6RS,trans) 6-(1-méthyléthyl) amino 7-hydroxy 4,5,6,7-tétrahydro imidazo [5,4,1-j-k][1] benzazépin-2-(1H)-one sera appelé produit $P_1$.

**Exemple 1 :**

On a préparé des comprimés renfermant :
Produit $P_1$ 5 mg
Excipient q.s. pour un comprimé terminé à 100 mg.
(détail de l'excipient : lactose, amidon de blé, amidon traité, amidon de riz, stéarate de magnésium, talc).

**Exemple 2 :**

On a préparé des granulés renfermant 25 mg de produit $P_1$ pour une dose journalière de granulés.

**Exemple 3 : Associations**

On a préparé des associations renfermant :
a) des granulés de produit $P_1$
25 mg de principe actif pour une dose journalière de granulés et,
b) des implants $P_2$ renfermant :
acétate de trembolone 20 mg
zéranol 36 mg.

**Exemple 4 :**

On a préparé des comprimés renfermant :
Salbutamol 5 mg
Excipient q.s. pour un comprimé terminé à 100 mg

(détail de l'excipient : lactose, amidon de blé, amidon traité, amidon de riz, stéarate de magnésium, talc).

**Exemple 5 :**

On a préparé des granulés renfermant 25 mg de salbutamol pour une dose journalière de granulés.

**Exemple 6 : Associations.**

On a préparé des granulés renfermant :
a) des granulés de salbutamol,
25 mg de principe actif pour une dose journalière de granulés et,
b) des implants renfermant acétate de trenbolone 20 mg
des implants renfermant du zéranol 36 mg

**Exemple 7 :**

L'essai a été effectué sur des porcs.
L'essai a été effectué sur des porcs.
Les animaux sont divisés en 4 lots :
- un lot témoin (ne recevant que la nourriture),
- un lot recevant 250 µg/kg de produit $P_1$ dans sa nourriture par jour,
- un lot recevant 250 µg/kg de produit $P_1$ dans sa nourriture par jour et auquel on a implanté un implant $P_2$ (voir exemple 3),
- un lot recevant uniquement l'implant $P_2$ en plus de sa nourriture.

Le produit $P_1$ est donné dans la nourriture,
le mélange $P_2$ est administré sous forme d'implant dans le tissu sous-cutané situé derrière l'oreille.
Tous les animaux recoivent la même nourriture pendant 90 jours.
Les résultats sont réunis dans le tableau suivant :

| | Témoins | Lot 1 | Lot 2 | Lot 3 |
|---|---|---|---|---|
| Nombre d'animaux | 10 | 10 | 10 | 10 |
| Nombre de jours moyen d'engraissement | 28 | 28 | 28 | 28 |
| Poids moyen à l'implantatition en KG | 82,00 | 81,80 | 81,80 | 81,80 |
| Poids moyen à l'abattage en KG | 103,00 | 103,90 | 107,60 | 103,30 |
| Gain de poids en KG | 21,00 | 22,10 | 25,80 | 21,50 |
| Gain de poids quotidien moyen en KG | 0,750 | 0,789 | 0,921 | 0,768 |
| **Conclusion :** 1) Tous les lots traités ont une croissance supérieure à celle du lot témoin. 2) Le lot qui a reçu le produit $P_1$ et l'implant $P_2$ a une croissance exceptionnelle très supérieure à l'addition des effets provoqués par les produits $P_1$ et $P_2$ administrés séparément. | | | | |

**Exemple 8 :**

L'essai a été effectué sur des porcs.
Les animaux sont divisés en 4 Lots :
- un lot recevant 0,25 mg/kg de salbutamol dans sa nourriture par jour,
- un lot recevant 0,25 mg/kg de salbutamol dans sa norriture par jour et auquel on a implanté un implant $P_2$ (voir exemple 3),
- un lot recevant uniquement l'implant $P_2$ en plus de sa nourriture.

Le salbutamol est donné dans la nourriture.
Le produit $P_2$ est administré sous forme d'implant dans le tissu sous-cutané situé derrière l'oreille.
Tous les animaux reçoivent la même nourriture pendant 90 jours.
Les résultats sont réunis dans le tableau suivant :

| traitements | témoins | zéranol 36 mg + acétate de trembolone 20mg | salbutamol 0,25 mg + zéranol 36 mg + acétate trembolone 20mg | salbutamol 0,25 mg |
|---|---|---|---|---|
| temps de traitement avant abattage en jours | | 54 | 54 | 54 |
| N¤ des lots | I | II | IV | V |
| Nombre animaux | 9 | 9 | 9 | 9 |
| Poids moyen le jour du traitement (kg) | 61,67 | 61,67 | 61,67 | 61,56 |
| Poids moyen à la fin de l'essai (kg) | 103,22 | 105,67 | 106,22 | 104,44 |
| Gain de poids moyen kg) | 41,56 | 44,00 | 44,56 | 42,89 |
| G.M.Q. (kg) Gain de poids | 0,770 | 0,815 | 0,825 | 0,794 |
| Poids des carcasses froides (kg) | 80,22 | 82,82 | 84,89 | 82,49 |
| Aliment consommé (kg) | 130,17 | 129,79 | 129,21 | 130,02 |
| Indice de consommation | 3,14 | 2,96 | 2,92 | 3,05 |
| **CONCLUSION** : Les associations de l'invention entraînent de très bons résultats d'élevage. | | | | |

**Exemple 9 :**

L'essai a été effectué sur des veaux.
Les animaux sont divisés en 2 lots de veaux de sexe mâle.
Les traitements ont été mis en place selon le schéma suivants :
- Lot I ou lot témoin : ne recevant aucun produit.
- Lot II : Lot recevant 0,1 mg/kg de poids vif de salbutamol.

L'expérimentation s'est déroulée en 3 temps :
- une période expérimentale de 85 jours,
- une période expérimentale de 34 jours,
- une période post expérimentale de 15 jours.

Les pesées sont effectuées tous les 15 jours.
Les animaux reçoivent une alimentation rationnée pendant toute la durée de l'essai.
Les résultats obtenus sont résumés dans le tableau suivant :

| N° des lots | I | II |
|---|---|---|
| Nombre d'animaux | 9 | 10 |
| Nombre de jours d'engraissement | 34 | 34 |
| Poids moyen le jour du traitement (kg) | 140,22 | 141,50 |
| Poids moyen à la fin de de l'essai (kg) | 179,00 | 185,40 |
| Gain de poids moyen (kg) | 38,78 | 43,90 |
| G.M.Q. (kg) | 1,141 | 1,291 |
| Aliments consommés (kg) | 81,90 | 82,00 |
| Indice de consommation | 2,11 | 1,87 |

**Exemple 10 :**

L'essai a été effectué sur des agneaux mâles entiers et des femelles.
Les animaux sont divisés en 3 lots :
- un lot recoit 0,2 mg/kg de produit $P_1$ (lot 1),
- un lot recoit 0,4 mg/kg de produit $P_1$ (lot 2),
- un lot témoin ne recoit aucun traitement.

Le produit $P_1$ est donné dans l'alimentation:
l'alimentation est constituée de granulés donnés en quantités rationnées, et de foin fourni à volonté.
On a constaté une nette amélioration des gains de poids et de la conformation des animaux des lots 1 et 2.

**Revendications**

**1.** Les associations caractérisées en ce qu'elles renferment :
a) une composition zootechnique renfermant un béta adrénergique,
b) une composition zootechnique renfermant comme principe actif, un stéroïde de formule (A) :

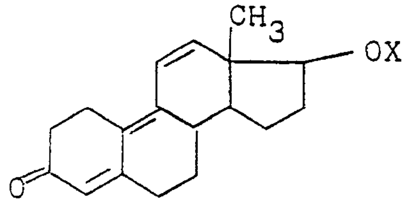

(A)

dans laquelle X représente un atome d'hydrogène, un radical alcoyle saturé ou insaturé renfermant de 1 à 6 atomes de carbone dont l'un des atomes de carbone peut être remplacé par un atome d'oxygène ou X représente un radical acyle dérivé d'un acide carboxylique renfermant de 1 à 18

atomes de carbone, pour une utilisation simultanée, séparée ou étalée dans le temps.

**2.** Les associations définies à la revendication 1 caractérisées en ce que le stéroïde de formule (A) est l'acétate de trenbolone ou 3-oxo 17béta acétoxy estra-4,9,11-triène.

**3.** Les associations définies à la revendication 1 ou 2 caractérisées en ce qu'elles renferment comme béta adrénergique au moins un composé de formule générale (I) :

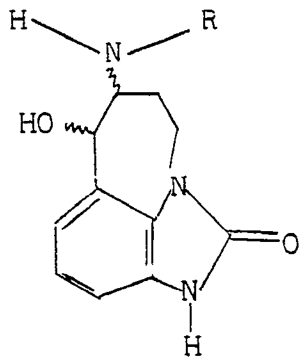

(I)

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phénnumyloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans ou un de ses sels d'addition avec les acides minéraux ou organiques.

**4.** Les associations définies à l'une quelconque des revendications 1 à 3, caractérisées en ce qu'elles renferment un produit de formule (I) dans laquelle R représente un atome d'hydrogène ou un radical alcoyle linéaire ou ramifié renfermant de 1 à 5 atomes de carbone ou un de ses sels d'addition avec les acides minéraux ou organiques.

**5.** Les associations caractérisées à l'une quelconque des revendications 1 à 4, caractérisées en ce qu'elles renferment :

- la (6RS, trans) 6-(1-méthyléthyl) amino 7-hydroxy 4,5,6,7-tétrahydro imidazo [5,4,1-j-k] [1] benzazépin-2-(1H)-one ainsi que ses sels d'addition avec les acides minéraux ou organiques et notamment son chlorhydrate.

**6.** Les associations définies à la revendication 1 ou 2 caractérisées en ce qu'elles renferment comme béta adrénergique le salbutamol de formule :

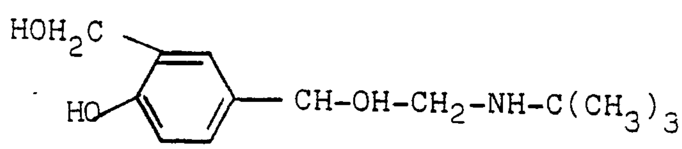

**7.** Les associations définies à la revendication 1 caractérisées en ce qu'elles renferment :

a) une composition zootechnique renfermant un béta adrénergique,

b) une composition zootechnique renfermant un produit de formule (A) telle que définie ci-dessus,

c) une composition zootechnique renfermant du zéranol, pour une utilisation simultanée, séparée ou étalée dans le temps.

**8.** Les associations définies à la revendication 1, caractérisées en ce qu'elles renferment :

a) une composition zootechnique renfermant un béta adrénergique,

b) une composition zootechnique renfermant un produit de formule (A),
c) une composition zootechnique renfermant de l'estradiol, pour une utilisation simultanée, séparée ou étalée dans le temps.

**9.** Les associations définies à la revendication 7 caractérisées en ce qu'elles renferment :
a) une composition zootechnique renfermant comme béta adrénergique, un produit de formule (I) défini comme précédemment,
b) une composition zootechnique renfermant comme principe actif un produit de formule (A) définie comme précédemment ;
c) une composition zootechnique renfermant comme principe actif du zéranol, pour une utilisation simultanée, séparée ou étalée dans le temps.

**10.** Les associations définies à la revendication 8, caractérisées en ce qu'elles renferment :
a) une composition zootechnique renfermant comme béta adrénergique un produit de formule (I) définie comme précédemment,
b) une composition zootechnique renfermant comme principe actif un produit de formule (A) définie comme précédemment,
c) une composition zootechnique renfermant comme principe actif de l'estradiol, pour une utilisation simultanée, séparée ou étalée dans le temps.

**11.** Les associations définies à l'une quelconque des revendications 7 à 10, caractérisées en ce que le produit de formule (I) est la (6RS,trans) 6-(1-méthyléthyl) amino 7-hydroxy 4,5,6,7-tétrahydro imidazo [5,4,1-j-k][1] benzazépin-2-(1H) one ou l'un de ses sels d'addition avec les acides organiques ou minéraux et le produit de formule (A) est l'acétate de trenbolone pour une utilisation simultanée, séparée ou étalée dans le temps.

**12.** Les associations définies à la revendication 7 caractérisées en ce qu'elles renferment :
a) une composition zootechnique renfermant comme béta adrénergique du salbutamol,
b) une composition zootechnique renfermant de l'acétate de trenbolone,
c) une composition zootechnique renfermant du zéranol, pour une utilisation simultanée, séparée ou étalée dans le temps.

**13.** Les associations définies à la revendication 8 caractérisées en ce qu'elles renferment :
a) une composition zootechnique renfermant comme béta adrénergique du salbutamol,
b) une composition zootechnique renfermant de l'acétate de trenbolone,
c) une composition zootechnique renfermant de l'estradiol, pour une utilisation simultanée, séparée ou étalée dans le temps.

**14.** Les associations définies à l'une quelconque des revendications 7 à 11, caractérisées en ce qu'elles renferment :
a) une composition zootechnique renfermant comme principe actif un produit de formule (I) définie comme précédemment,
b) une composition zootechnique renfermant comme principes actifs :
$b_1$) un produit de formule (A) défini comme précédemment,
$b_2$) du zéranol ou de l'estradiol,
pour une utilisation simultanée séparée ou étalée dans le temps.

**15.** Les associations définies à la revendication 14, caractérisées en ce qu'elles renferment :
a) une composition zootechnique renfermant comme principe actif la (6RS,trans) 6-(1-méthyléthyl) amino 7-hydroxy 4,5,6,7-tétrahydro imidazo [5,4,1-j-k][1]benzazépin-2-(1H)-one ou l'un de ses sels d'addition avec les acides organiques ou minéraux,
b) une composition zootechnique renfermant comme principes actifs :
$b_1$) l'acétate de trenbolone,
$b_2$) le zéranol,
pour une utilisation simultanée, séparée ou étalée dans le temps.

**16.** Les associations définies à la revendication 7 ou 12, caractérisées en ce qu'elles renferment :
a) une composition zootechnique renfermant du salbutamol,

b) une composition zootechnique renfermant :
$b_1$) de l'acétate de trenbolone et,
$b_2$) du zéranol,
pour une utilisation simultanée, séparée ou étalée dans le temps.

**17.** Les associations définies à la revendication 8 ou 13, caractérisées en ce qu'elles renferment :
a) une composition zootechnique renfermant du salbutamol,
b) une composition zootechnique renfermant :
$b_1$) de l'acétate de trenbolone et,
$b_2$) de l'estradiol,
pour une utilisation simultanée, séparée ou étalée dans le temps.

**18.** Les compositions zootechniques renfermant comme principe actif au moins un composé de formule générale (I) :

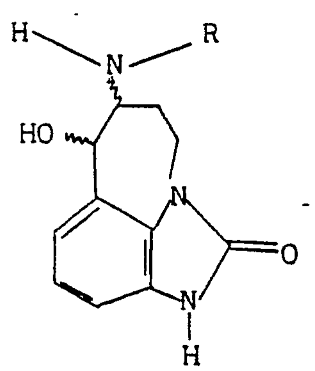

(I)

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans ou un de ses sels d'addition avec les acides minéraux ou organiques.

**19.** Les compositions zootechniques définies à la revendication 18 renfermant comme principe actif au moins un composé de formule I dans laquelle R représente un atome d'hydrogène ou un radical alcoyle linéaire ou ramifié renfermant de 1 à 5 atomes de carbone ou un de ses sels d'addition avec les acides minéraux ou organiques.

**20.** Les compositions zootechniques renfermant comme principe actif:
- la (6RS, trans) 6-(1-méthyléthyl) amino 7-hydroxy 4,5,6,7-tétrahydro imidazo /5,4,1-j-k/ /1/ benzazépin-2-(1H)-one ainsi que ses sels d'addition avec les acides minéraux ou organiques et notamment son chlorhydrate.

**21.** Les compositions zootechniques renfermant comme principe actif du salbutamol.

**22.** Les associations telles que définies à l'une quelconque des revendications 1 à 17, caractérisées en ce qu'elles sont destinées à l'administration chez le porc.

**23.** Les associations telles que définies à l'une quelconque des revendications 1 à 17, caractérisées en ce qu'elles sont destinées à l'administration chez le veau.

**24.** Les compositions zootechniques telles que définies à l'une quelconque des revendications 18 à 20, caractérisées en ce qu'elles sont destinées à l'administration chez le porc.

**25.** Les compositions zootechniques telles que définies à l'lune quelconque des revendications 18 à 20,

caractérisées en ce qu'elles sont destinées à l'administration chez le veau.

**26.** Les compositions zootechniques telles que définies à la revendication 21, caractérisées en ce qu'elles sont destinées à l'administration chez le porc.

**27.** Les compositions zootechniques telles que définies à la revendication 21, caractérisées en ce qu'elles sont destinées à l'administration chez le veau.

**28.** Les associations et compositions zootechniques telles que définies à l'une quelconque des revendications 1 à 21, caractérisées en ce qu'elles sont destinées à l'administration chez la volaille.

**29.** Les associations et compositions zootechniques telles que définies à l'une quelconque des revendications 1 à 28, caractérisées en ce qu'elles sont incorporées à des mélanges nutritiels adaptés à l'alimentation animale.

**30.** Les associations et compositions zootechniques telles que définies à l'une quelconque des revendications 1 à 29, caractérisées en ce qu'elles sont destinées à l'administration par voie orale à raison de 10 à 1000 $\mu$g de béta adrénergique par kg de poids d'animal et par jour.

**31.** Les associations et compositions zootechniques telles que définies à l'une quelconque des revendications 1 à 30, caractérisées en ce qu'elles renferment de 1 à 300 mg de béta adrénergique.

**Claims**

**1.** The combinations characterized in that they contain:
a) a zootechnic composition containing a beta adrenergic,
b) a zootechnic composition containing, as active ingredient, a steroid of formula (A):

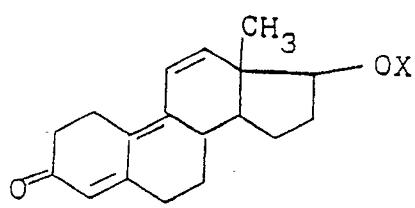

(A)

in which X represents a hydrogen atom, a saturated or unsaturated alkyl radical containing 1 to 6 carbon atoms of which one of the carbon atoms can be replaced by an oxygen atom or X represents an acyl derivative of a carboxylic acid containing 1 to 18 carbon atoms, for simultaneous or separate use or for use spread out over time.

**2.** The combinations defined in claim 1 characterized in that the steroid of formula (A) is trenbolone acetate or 3-oxo-17beta-acetoxy-estra-4,9,11-triene.

**3.** The combinations defined in claim 1 or 2 characterized in that they contain as beta adrenergic at least one compound of general formula (I):

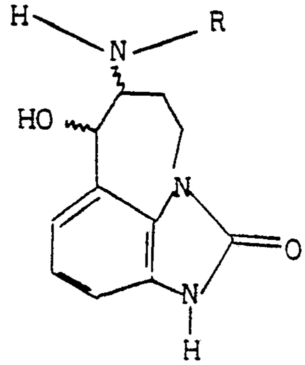

(I)

in which R represents a hydrogen atom, a linear or branched alkyl radical containing 1 to 8 carbon atoms, optionally substituted by a hydroxy radical, by a phenyl radical or by a phenyloxy radical or a cycloalkyl radical containing 3 to 7 carbon atoms, or a 4-piperidinyl radical the nitrogen atom of which optionally carries an alkyl substituent containing 1 to 4 carbon atoms and the dotted lines signify that the hydroxyl in position 7 and the amino radical in position 6 are of trans configuration or one of its addition salts with mineral or organic acids.

**4.** The combinations defined in any one of claims 1 to 3, characterized in that they contain a product of formula (I) in which R represents a hydrogen atom or a linear or branched alkyl radical containing 1 to 5 carbon atoms or one of its addition salts with mineral or organic acids.

**5.** The combinations characterized in any one of claims 1 to 4, characterized in that they contain:
- (6RS, trans)-6-(1-methylethyl)-amino-7-hydroxy-4,5,6,7-tetrahydro imidazo-[5,4,1-j-k]-[1]-benzazepin-2-(1H)-one as well as its addition salts with mineral or organic acids and notably its hydrochloride.

**6.** The combinations defined in claim 1 or 2 characterized in that they contain as beta adrenergic, the salbutamol of formula:

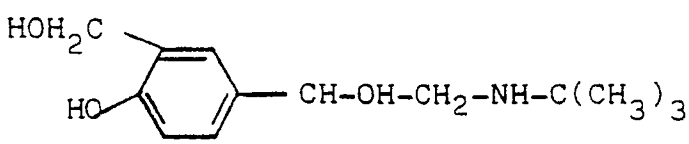

**7.** The combinations defined in claim 1, characterized in that they contain:
a) a zootechnic composition containing a beta adrenergic,
b) a zootechnic composition containing a product of formula (A) as defined above,
c) a zootechnic composition containing zeranol, for simultaneous or separate use or for use spread out over time.

**8.** The combinations defined in claim 1, characterized in that they contain:
a) a zootechnic composition containing a beta adrenergic,
b) a zootechnic composition containing a product of formula (A),
c) a zootechnic composition containing estradiol, for simultaneous or separate use or for use spread out over time.

**9.** The combinations defined in claim 7 characterized in that they contain:
a) a zootechnic composition containing as beta adrenergic, a product of formula (I) as defined previously,
b) a zootechnic composition containing, as active ingredient, a product of formula (A) as defined previously,
c) a zootechnic composition containing, as active ingredient, zeranol, for simultaneous or separate use or for use spread out over time.

**10.** The combinations defined in claim 8, characterized in that they contain:

a) a zootechnic composition containing as beta adrenergic, a product of formula (I) as defined previously,

b) a zootechnic composition containing, as active ingredient, a product of formula (A) as defined previously,

c) a zootechnic composition containing, as active ingredient, estradiol, for simultaneous or separate use or for use spread out over time.

**11.** The combinations defined in any one of claims 7 to 10, characterized in that the product of formula (I) is (6RS, trans)-6-(1-methylethyl)-amino-7-hydroxy-4,5,6,7-tetrahydro imidazo-[5,4,1-j-k]-[1]-benzazepin-2-(1H)-one or one of its addition salts with mineral or organic acids and the product of formula (A) is trenbolone acetate for simultaneous or separate use or for use spread out over time.

**12.** The combinations defined in claim 7, characterized in that they contain:

a) a zootechnic composition containing salbutamol as beta adrenergic,

b) a zootechnic composition containing trenbolone acetate,

c) a zootechnic composition containing zeranol, for simultaneous or separate use or for use spread out over time.

**13.** The combinations defined in claim 8 characterized in that they contain:

a) a zootechnic composition containing salbutamol as beta adrenergic,

b) a zootechnic composition containing trenbolone acetate,

c) a zootechnic composition containing estradiol, for simultaneous or separate use or for use spread out over time.

**14.** The combinations defined in any one of claims 7 to 11, characterized in that they contain:

a) a zootechnic composition containing as active ingredient a product of formula (I) as defined previously,

b) a zootechnic composition containing as active ingredients:

$b_1$) a product of formula (A) as defined previously,

$b_2$) zeranol or estradiol,

for simultaneous or separate use or use spread out over time.

**15.** The combinations defined in claim 14, characterized in that they contain:

a) a zootechnic composition containing as active ingredient (6RS, trans)-6-(1-methylethyl)-amino-7-hydroxy-4,5,6,7-tetrahydro imidazo-[5,4,1-j-k]-[1]-benzazepin-2-(1H)-one or one of its addition salts with organic or mineral acids, b) a zootechnic composition containing as active ingredients:

$b_1$) trenbolone acetate,

$b_2$) zeranol,

for simultaneous or separate use or for use spread out over time.

**16.** The combinations defined in claim 7 or 12, characterized in that they contain:

a) a zootechnic composition containing salbutamol,

b) a zootechnic composition containing:

$b_1$) trenbolone acetate and,

$b_2$) zeranol,

for simultaneous or separate use or for use spread out over time.

**17.** The combinations defined in claim 8 or 13, characterized in that they contain :

a) a zootechnic composition containing salbutamol,

b) a zootechnic composition containing:

$b_1$) trenbolone acetate and,

$b_2$) estradiol,

for simultaneous or separate use or for use spread out over time.

**18.** The zootechnic compositions containing as active ingredient at least one compound of general formula (I):

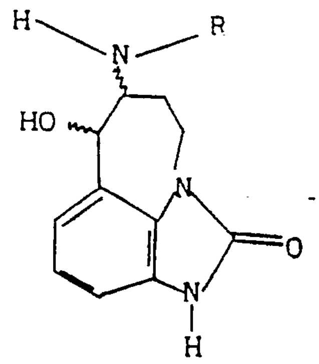

(I)

in which R represents a hydrogen atom, a linear or branched alkyl radical containing 1 to 8 carbon atoms, optionally substituted by a hydroxy radical, by a phenyl radical or by a phenyloxy radical or a cycloalkyl radical containing 3 to 7 carbon atoms, or a 4-piperidinyl radical the nitrogen atom of which optionally carries an alkyl substituent containing 1 to 4 carbon atoms and the wavy lines signify that the hydroxyl in position 7 and the amino radical in position 6 are of trans configuration or one of its addition salts with mineral or organic acids.

**19.** The zootechnic compositions defined in claim 18 containing, as active ingredient, at least one compound of formula (I) in which R represents a hydrogen atom or a linear or branched alkyl radical containing 1 to 5 carbon atoms or one of its addition salts with mineral or organic acids.

**20.** The zootechnic compositions containing as active ingredient:
(6RS, trans)-6-(1-methylethyl)-amino-7-hydroxy-4,5,6,7-tetrahydro imidazo-[5,4,1-j-k]-[1]-benzazepin-2-(1H)-one as well as its addition salts with mineral or organic acids and notably its hydrochloride.

**21.** The zootechnic compositions containing salbutamol as active ingredient.

**22.** The combinations as defined in any one of claims 1 to 17, characterized in that they are intended for administration to pigs.

**23.** The combinations as defined in any one of claims 1 to 17, characterized in that they are intended for administration to calves.

**24.** The zootechnic compositions as defined in any one of claims 18 to 20, characterized in that they are intended for administration to pigs.

**25.** The zootechnic compositions as defined in any one of claims 18 to 20, characterized in that they are intended for administration to calves.

**26.** The zootechnic compositions as defined in claim 21, characterized in that they are intended for administration to pigs.

**27.** The zootechnic compositions as defined in claim 21, characterized in that they are intended for administration to calves.

**28.** The zootechnic combinations and compositions as defined in any one of claims 1 to 21, characterized in that they are intended for administration to poultry.

**29.** The zootechnic combinations and compositions as defined in any one of claims 1 to 28, characterized in that they are incorporated in food mixtures suitable for animal fodder.

**30.** The zootechnic combinations and compositions as defined in any one of claims 1 to 29, characterized in that they are intended for administration by oral route at the rate of 10 to 1,000 g of beta adrenergic per kg of animal weight and per day.

**31.** The zootechnic combinations and compositions as defined in any one of claims 1 to 30, characterized in that they contain from 1 to 300 mg of beta adrenergic.

**Patentansprüche**

**1.** Assoziationen, dadurch gekennzeichnet, daß sie enthalten:
a) eine zootechnische Zusammensetzung, die ein ß-Adrenergikum enthält;
b) eine zootechnische Zusammensetzung, die als Wirkstoff ein Steroid der Formel (A)

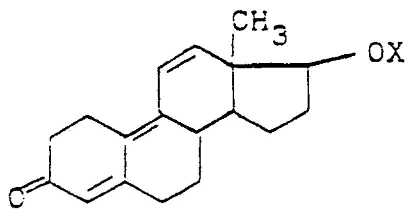

(A)

worin X für ein Wasserstoffatom, einen gesättigten oder ungesättigten Alkylrest mit 1 bis 6 Kohlenstoffatomen, wovon eines der Kohlenstoffatome durch ein Sauerstoffatom ersetzt sein kann, steht oder X einen sich von einer Carbonsäure mit 1 bis 18 Kohlenstoffatomen ableitenden Acylrest wiedergibt, für eine gleichzeitige, getrennte oder zeitlich verschobene Anwendung enthält.

**2.** Assoziationen gemäß Anspruch 1, dadurch gekennzeichnet, daß das Steroid der Formel A das Trenbolonacetat oder 3-Oxo-17ß-acetoxyöstra-4,9,11-trien ist.

**3.** Assoziationen gemäß Anspruch 1 oder 2, dadurch gekennzeichnet, daß sie als ß-Adrenergikum zumindest eine Verbindung der allgemeinen Formel (I)

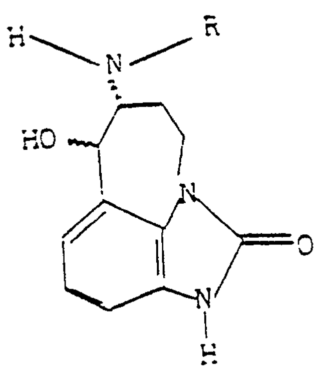

(I)

worin R ein Wasserstoffatom, einen linearen oder verzweigten Alkylrest mit 1 bis 8 Kohlenstoffatomen, welcher gegebenenfalls durch einen Hydroxyrest, durch einen Phenylrest oder durch einen Phenyloxyrest substituiert ist oder einen Cycloalkylrest mit 3 bis 7 Kohlenstoffatomen oder einen 4-Piperidinylrest, dessen Stickstoffatom gegebenenfalls einen Alkylsubstituenten mit 1 bis 4 Kohlenstoffatomen enthält, bedeutet und die gewellten Linien anzeigen, daß die Hydroxylgruppe in 7-Stellung und die Aminogruppe in 6-Stellung in trans-Konfiguration vorliegen, oder eines ihrer Additionssalze mit Mineral- oder organischen Säuren enthalten.

**4.** Assoziationen gemäß einem der Ansprüche 1 bis 3, dadurch gekennzeichnet, daß sie ein Produkt der Formel (I), worin R für ein Wasserstoffatom oder einen linearen oder verzweigten Alkylrest mit 1 bis 5 Kohlenstoffatomen steht, oder eines seiner Additionssalze mit Mineral- oder organischen Säuren enthalten.

**5.** Assoziationen gekennzeichnet gemäß einem der Ansprüche 1 - 4, dadurch gekennzeichnet, daß sie enthalten :

das(6RS,trans)-6-(1-Methylethyl)-amino-7-hydroxy-4,5,6,7-tetrahydro-imidazo-[5,4,1-j-k]-[1]-benzazepin-2-(1H)-on sowie seine Additionssalze mit mineralischen oder organischen Säuren und insbesondere dessen Hydrochlorid.

**6.** Assoziationen gemäß Anspruch 1 oder 2, dadurch gekennzeichnet, daß sie als ß-Adrenergikum das Salbutamol der Formel

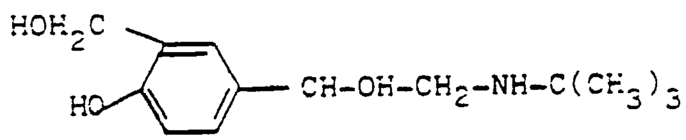

enthalten.

**7.** Assoziationen gemäß Anspruch 1, dadurch gekennzeichnet, daß sie enthalten:
a) eine zootechnische Zusammensetzung, die ein ß-Adrenergikum enthält,
b) eine zootechnische Zusammensetzung, die ein Produkt der Formel (A), wie vorstehend definiert, enthält,
c) eine zootechnische Zusammensetzung, die Zeranol enthält, für eine gleichzeitige, getrennte oder zeitlich verschobene Verwendung.

**8.** Assoziationen gemäß Anspruch 1, dadurch gekennzeichnet, daß sie enthalten:
a) eine zootechnische Zusammensetzung, die ein ß-Adrenergikum enthält,
b) eine zootechnische Zusammensetzung, die ein Produkt der Formel (A) enthält,
c) eine zootechnische Zusammensetzung, die Östradiol enthält, für eine gleichzeitige, getrennte oder zeitlich verschobene Verwendung.

**9.** Assoziationen gemäß Anspruch 7, dadurch gekennzeichnet, daß sie enthalten:
a) eine zootechnische Zusammensetzung, die als ß-Adrenergikum ein Produkt der Formel (I), wie vorstehend definiert, enthält,
b) eine zootechnische Zusammensetzung, die als Wirkstoff ein Produkt der Formel (A), wie vorstehend definiert, enthält,
c) eine zootechnische Zusammensetzung, die als Wirkstoff Zeranol enthält, für eine gleichzeitige, getrennte oder zeitlich verschobene Verwendung.

**10.** Assoziationen gemäß Anspruch 8, dadurch gekennzeichnet, daß sie enthalten:
a) eine zootechnische Zusammensetzung, die als ß-Adrenergikum ein Produkt der Formel (I), wie vorstehend definiert, enthält,
b) eine zootechnische Zusammensetzung, die als Wirkstoff ein Produkt der Formel (A), wie vorstehend definiert, enthält,
c) eine zootechnische Zusammensetzung, die als Wirkstoff Östradiol enthält, für eine gleichzeitige, getrennte oder zeitlich verschobene Verwendung.

**11.** Assoziationen gemäß einem der Ansprüche 7 bis 10, dadurch gekennzeichnet, daß das Produkt der Formel (I) (6RS,trans)-6-(1-Methylethyl)-amino-7-hydroxy-4,5,6,7-tetrahydro-imidazo-[5,4,1-j-k]-[1]-benzazepin-2-(1H)-on oder eines seiner Additionssalze mit organischen oder Mineralsäuren ist und das Produkt der Formel (A) Trenbolonacetat ist, für eine gleichzeitige, getrennte oder zeitliche verschobene Verwendung.

**12.** Assoziationen gemäß Anspruch 7, dadurch gekennzeichnet, daß sie enthalten:
a) eine zootechnische Zusammensetzung, welche als ß-Adrenergikum Salbutamol enthält,
b) eine zootechnische Zusammensetzung, die Trenbulonacetat enthält,
c) eine zootechnische Zusammensetzung, die Zeranol enthält, für eine gleichzeitige, getrennte oder zeitlich verschobene Verwendung.

**13.** Assoziationen gemäß Anspruch 8, dadurch gekennzeichnet, daß sie enthalten:
a) eine zootechnische Zusammensetzung, die als ß-Adrenergikum Salbutamol enthält,
b) eine zootechnische Zusammensetzung, die Trenbolonacetat enthält,

c) eine zootechnische Zusammensetzung, die Östradiol enthält, für eine gleichzeitige, getrennte oder zeitlich verschobene Verwendung.

**14.** Assoziationen gemäß einem der Ansprüche 7 bis 11, dadurch gekennzeichnet, daß sie enthalten:
a) eine zootechnische Zusammensetzung, die als Wirkstoff ein Produkt der Formel (I), wie vorstehend definiert, enthält,
b) eine zootechnische Zusammensetzung, die als Wirkstoffe
$b_1$) ein Produkt der Formel (A), wie vorstehend definiert,
$b_2$) Zeranol oder Östradiol enhält,
für eine gleichzeitige, getrennte oder zeitlich verschobene Verwendung.

**15.** Assoziationen gemäß Anspruch 14, dadurch gekennzeichnet, daß sie enthalten:
a) eine zootechnische Zusammensetzung, die als Wirkstoff das (6RS,trans)-6-(1-Methylethyl)-amino-7-hYdroxy-4,5,6,7-tetrahydro-imidazo-[5,4,1-j-k]-[1]-benzazepin-2-(1H)-on oder eines seiner Additionssalze mit organischen oder Mineralsäuren enthält,
b) eine zootechnische Zusammensetzung, die als Wirkstoffe
$b_1$) Trenbolonacetat,
$b_2$) Zeranol enthält,
für eine gleichzeitige, getrennte oder zeitlich verschobene Verwendung.

**16.** Assoziationen gemäß Anspruch 7 oder 12, dadurch gekennzeichnet, daß sie enthalten:
a) eine zootechnische Zusammensetzung, die Salbutamol enthält,
b) eine zootechnische Zusammensetzung, die enthält:
$b_1$) Trenbolonacetat und
$b_2$) Zeranol
für eine gleichzeitige, getrennte oder zeitlich verschobene Verwendung.

**17.** Assoziationen gemäß Anspruch 8 oder 13, dadurch gekennzeichnet, daß sie enthalten:
a) eine zootechnische Zusammensetzung, die Salbutamol enthält,
b) eine zootechnische Zusammensetzung, die enthält:
$b_1$) Trenbolonacetat und
$b_2$) Östradiol,
für eine gleichzeitige, getrennte oder zeitlich verschobene Verwendung.

**18.** Zootechnische Zusammensetzungen, enthaltend als Wirkstoff zumindest eine Verbindung der allgemeinen Formel (I)

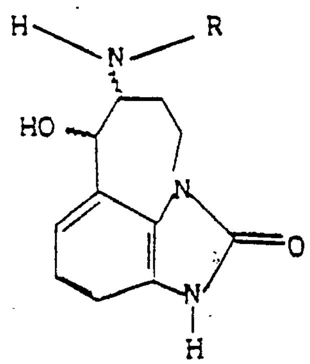

(I)

worin R ein Wasserstoffatom, einen linearen oder verzweigten Alkylrest mit 1 bis 8 Kohlenstoffatomen, der gegebenenfalls durch einen Hydroxyrest, durch eine Phenylgruppe oder durch einen Phenyloxyrest substituiert ist, oder einen Cycloalkylrest mit 3 bis 7 Kohlenstoffatomen oder einen 4-Piperidinylrest, dessen Stickstoffatom gegebenenfalls einen Alkylsubstituenten mit 1 bis 4 Kohlenstoffatomen enthält, bedeutet und die gewellten Linien anzeigen, daß die Hydroxylgruppe in 7-Stellung und die Aminogruppe in 6-Stellung in trans-Konfiguration vorliegen oder eines ihrer Additionssalze mit Mineral- oder organischen Säuren.

**19.** Zootechnische Zusammensetzungen gemäß Anspruch 18, enthaltend als Wirkstoff zumindest eine Verbindung der Formel (I), worin R für ein Wasserstoffatom oder einen linearen oder verzweigten Alkylrest mit 1 bis 5 Kohlenstoffatomen steht, oder eines seiner Additionssalze mit Mineral- oder organischen Säuren.

**20.** Zootechnische Zusammensetzungen, enthaltend als Wirkstoff

das (6RS,trans)-6-(1-Methylethyl)-amino-7-hydroxy-4,5,6,7-tetrahydro-imidazo-[5,4,1-j-k]-[1]-benzazepin-2-(1H)-on sowie dessen Additionssalze mit Mineral- oder organischen Säuren und insbesondere dessen Hydrochlorid.

**21.** Zootechnische Zusammensetzungen, enthaltend als Wirkstoff Salbutamol.

**22.** Assoziationen gemäß einem der Ansprüche 1 bis 17, dadurch gekennzeichnet, daß sie für die Verabreichung an das Schwein bestimmt sind.

**23.** Assoziationen gemäß .einem der Ansprüche 1 bis 17, dadurch gekennzeichnet, daß sie für die Verabreichung an das Kalb bestimmt sind.

**24.** Zootechnische Zusammensetzungen gemäß einem der Ansprüche 18 bis 20, dadurch gekennzeichnet, daß sie für die Verabreichung an das Schwein bestimmt sind.

**25.** Zootechnische Zusammensetzungen gemäß einem der Ansprüche 18 bis 20, dadurch gekennzeichnet, daß sie für die Verabreichung an das Kalb bestimmt sind.

**26.** Zootechnische Zusammensetzungen gemäß Anspruch 21, dadurch gekennzeichnet, daß sie für die Verabreichung an das Schwein bestimmt sind.

**27.** Zootechnische Zusammensetzungen gemäß Anspruch 21, dadurch gekennzeichnet, daß sie für die Verabreichung an das Kalb bestimmt sind.

**28.** Assoziationen und zootechnische Zusammensetzungen gemäß einem der Ansprüche 1 bis 21, dadurch gekennzeichnet, daß sie für die Verabreichung an das Geflügel bestimmt sind.

**29.** Zootechnische Assoziationen und Zusammensetzungen gemäß einem der Ansprüche 1 bis 28, dadurch gekennzeichnet, daß sie für die tierische Ernährung geeigneten Nährgemischen einverleibt sind.

**30.** Zootechnische Assoziationen und Zusammensetzungen gemäß einem der Ansprüche 1 bis 29, dadurch gekennzeichnet, daß sie für die orale Verabreichung in einer Menge von 10 bis 1000 g ß-Adrenergikum je kg Tierkörpergewicht und je Tag bestimmt sind.

**31.** Zootechnische Assoziationen und Zusammensetzungen gemäß einem der Ansprüche 1 bis 30, dadurch gekennzeichnet, daß sie 1 bis 300 mg ß-Adrenergikum enthalten.